**Europäisches Patentamt**

**European Patent Office**

**Office européen des brevets**

(19)

(11) Veröffentlichungsnummer : **0 259 398 B1**

(12) **EUROPÄISCHE PATENTSCHRIFT**

(45) Veröffentlichungstag der Patentschrift :
**15.01.92 Patentblatt 92/03**

(51) Int. Cl.⁵ : **A61F 9/00**

(21) Anmeldenummer : **87901386.0**

(22) Anmeldetag : **09.03.87**

(86) Internationale Anmeldenummer :
**PCT/DE87/00103**

(87) Internationale Veröffentlichungsnummer :
**WO 87/05205 11.09.87 Gazette 87/20**

(54) **VORRICHTUNG ZUR BEOBACHTUNG UND LASERBEHANDLUNG DES AUGES.**

(30) Priorität : **08.03.86 DE 3607721**
**08.11.86 DE 3638226**

(43) Veröffentlichungstag der Anmeldung :
**16.03.88 Patentblatt 88/11**

(45) Bekanntmachung des Hinweises auf die
Patenterteilung :
**15.01.92 Patentblatt 92/03**

(84) Benannte Vertragsstaaten :
**AT CH DE FR GB IT LI**

(56) Entgegenhaltungen :
**EP-A- 0 139 941**
**EP-A- 0 145 563**
**EP-A- 0 191 688**
**DE-A- 3 306 981**
**DE-A- 3 425 975**
**DE-A-31 487 48**
**US-A- 4 331 132**
**US-A-42 136 78**
**US-A-44 430 75**

(73) Patentinhaber : **G. RODENSTOCK**
**INSTRUMENTE GMBH**
**Drachenseestrasse 10-12**
**W-8000 München 70 (DE)**

(72) Erfinder : **FEUERSTEIN, Manfred**
**Gottfried-Böhm-Ring 23**
**W-8000 München 70 (DE)**
Erfinder : **KLINGBEIL, Ulrich**
**Daglfingerstr. 108**
**W-8000 München 81 (DE)**
Erfinder : **LANGOSCH, Herbert**
**Thalkirchnerstr. 282**
**W-8000 München (DE)**
Erfinder : **REIS, Werner**
**Hilblestr. 40**
**W-8000 München 19 (DE)**
Erfinder : **WILMS, Karl-Heinz**
**Hans-Bierlingstr. 47**
**W-8080 Emmering (DE)**
Erfinder : **EISENTRÄGER, Wolfgang**
**Prätoriusweg 6**
**W-8000 München 83 (DE)**
Erfinder : **BISLE, Werner**
**Karwendelstr. 21**
**W-8027 Neuried (DE)**
Erfinder : **PLESCH, Anton**
**Schinkelstrasse 1**
**W-8000 Munchen 40 (DE)**

(74) Vertreter : **Münich, Wilhelm, Dr.**
**Kanzlei Münich, Steinmann, Schiller**
**Willibaldstrasse 36/38**
**W-8000 München 21 (DE)**

EP 0 259 398 B1

## Beschreibung

## Technisches Gebiet

Die Erfindung bezieht sich auf ein Vorrichtung zur Beobachtung und Laserbehandlung des Auges gemäß dem Oberbegriff des Patentanspruchs 1.

## Stand der Technik

Bei der Beobachtung der hinteren Augenabschnitte besteht die Schwierigkeit, daß die Beleuchtung und die Beobachtung durch die Augenpupille und die häufig optisch nicht klaren vorderen Augenmedien erfolgen muß, an denen Reflexe auftreten, und die Abbildungsfehler erzeugen.

Deshalb ist bereits mehrfach vorgeschlagen worden, den Augenhintergrund nicht großflächig auszuleuchten, sondern mit auf einen möglichst kleinen Fleck fokussierten Beleuchtungslicht abzutasten und das reflektierte Licht in Zuordnung zur Abtastsequenz zu erfassen. Hierzu wird beispielsweise auf "The foundations of Ophthalmology", Bd. VII, S.307/308, Jg. 1962, die US-A-4 213 678 sowie die japanischen Patentveröffentlichungen 61-5730 und 50-138822 verwiesen.

Die aus den genannten Fundstellen bekannten Vorrichtungen unterscheiden sich u.a. in der Pupillenseparation: So werden in der japanischen Patentveröffentlichung 61-5730 eine "GULLSTRAND-Pupille", in der US-A-4 213 678 eine invertierte "GULLSTRAND-Pupille" und in der japanischen Patentveröffentlichung 50-138822 nebeneinanderliegende Pupillen für das Beleuchtungs- und das Beobachtungslicht vorgeschlagen.

Ferner wird in der EP-A-0 145 563 eine Vorrichtung zur Beobachtung der hinteren Augenabschnitte beschrieben, bei der sowohl der Beleuchtungs- als auch der Beobachtungslichtstrahl über die Abtasteinrichtung geführt. Ein derartiges "Double-Scanning-System" hat den Vorteil, daß der reflektierte Lichtstrahl mit einem ortsfesten Detektor mit vergleichsweise kleiner Fläche nachgewiesen werden kann.

Die gute Bildqualität, die diese bekannten Vorrichtungen liefern, prädestiniert diese Vorrichtungen auch zur Laserbehandlung des Auges, wie dies bereits in der US-A-4 213 678 vorgeschlagen worden ist. Von der in dieser Druckschrift beschriebenen Vorrichtung wird im übrigen bei der Formulierung des Oberbegriffs des Patentanspruchs 1 ausgegangen.

Obgleich die vorstehend genannten Vorrichtungen ein gegenüber herkömmlichen Ophthalmoskopen sehr gutes Bild des Augenhintergrundes liefern, konnten sie sich aus folgendem Grund noch nicht als Grundgeräte für die Laserbehandlung durchsetzen:

Die Bedienungsperson muß nicht nur die zu behandelnde Stelle wiederfinden, sondern auch den Laser bei Bewegungen des Auges nachführen. Diese auch bei anderen Laserbehandlungsvorrichtungen erforderlichen Tätigkeiten werden jedoch bei Vorrichtungen gemäß dem Oberbegriff des Anspruchs 1 zusätzlich dadurch erschwert, daß das auf dem Monitor wiedergegebenen Bild in der Regel monochrom und damit für den Augenarzt ungewohnt ist, da das Auge mit Laserlicht beleuchtet ist. Hierdurch wird das Identifizieren von Strukturen zusätzlich erschwert.

Andererseits ist in der DE-OS-34 25 975 eine Vorrichtung anderer Gattung vorgeschlagen worden, bei der eine Projektionseinrichtung vorgesehen ist, die insbesondere ein Angiogramm des zu behandelnden Auges in den Beobachtungsstrahlengang eines herkömmlichen ophthalmologischen Geräts, wie beispielsweise eine Spaltlampe einspiegelt.

Aber auch bei der aus der DE-OS-34 25 975 bekannten Vorrichtung muß die Bedienungsperson nicht nur das eingespiegelte Bild mit dem tatsächlich beobachteten Bild des zu behandelnden Auges zur Deckung bringen, sondern auch laufend den Laser "nachführen", wenn sich das Auge der zu behandelnden Person auch nur geringfügig bewegt hat. Lediglich das Identifizieren der zu behandelnden Stelle wird erleichtert.

Es ist ohne weiteres einzusehen, daß die Bedienungsperson durch die beiden Vorgänge - Nachführen des Beobachtungsstrahlengangs beispielweise einer Spaltlample, um das Angiogramm mit dem Fundusbild in Deckung zu bringen, Nachführen des Lasers auf die zu koagulierende Stelle - stark belastet ist. Eine derartige Belastung kann unter Umständen zu Fehlbedienungen und damit zu nachteiligen Behandlungsergebnissen führen.

Ferner ist eine Vorrichtung, bei der ein indirektes Ophthalmoskop verwendet wird, vorgeschlagen worden, bei der eine Steuereinheit vorgesehen ist, die den Laserstrahl bei Bewegungen des Auges nachführt (ARVO-Abstracts, Band 38, Zusammenfassung Nr. 92). Bei dieser bekannten Vorrichtung ist eine Bildverarbeitungseinheit vorgesehen, die ein von einer Bilderfassungseinrichtung während der Behandlung geliefertes Bild analysiert. Das Nachführen erfolgt bei dieser bekannten Vorrichtung dergestalt, daß die Bedienungsperson einen markanten Bereich, beispielsweise eine Blutgefäßverzweigung auswählt, und die Steuereinheit den Laserstrahl so nachführt, daß die Abstandsbeziehung der zu behandelnden Stelle zu diesem markanten Bereich während der Behandlung konstant bleibt. Der Grund für diese Vorgehensweise ist, daß die gegenwärtig zu in der ärztlichen Praxis tragbaren Gestehungskosten realisierbaren Bildverarbeitungseinheiten nicht in der Lage sind, beispielsweise den gesamten Augenhintergrund in "Echtzeit" zu analysieren, was zur Realisierung des Nachführens unter Berücksichtigung des gesamten erfaßten Bereichs erforderlich wäre.

Bei dieser bekannten Vorrichtung wird jedoch nicht berücksichtigt, daß es in einem menschlichen Auge im Rahmen der Auflösung einer typischen Bildverarbeitungseinheit "morphologisch" ähnliche Bereiche, beispielsweise "strukturell gleiche" Blutgefäßverzweigungen geben kann. Bei einer schnellen Augenbewegung ist es deshalb möglich, daß die Steuereinheit eine andere "morphologisch ähnliche" Blutgefäßverzweigung als den anfänglich ausgewählten Bereich erfaßt und diesen Bereich fälschlich als den Bereich identifiziert, zu dem die Abstandsbeziehung des Lasers konstant gehalten werden soll. Tritt dieser Fall auf, so richtet die Steuereinheit den Laser auf einen anderen Bereich als den zu behandelnden Bereich aus. Es bedarf keiner näheren Erläuterung, daß es dann bei dieser bekannten Vorrichtung zu unannehmbaren Schädigungen des Auges kommen kann.

## Darstellung der Erfindung

Der Erfindung liegt die Aufgabe zugrunde, ein Vorrichtung gemäß dem Oberbegriff des Patentanspruchs 1 derart weiterzubilden, daß einerseits die Bedienung erleichtert und andererseits Fehlerquellen durch fehlerhafte Ausrichtung des Lasers etc. ausgeschlossen werden.

Eine erfindungsgemäße Lösung dieser Aufgabe ist mit ihren Weiterbildungen in den Patentansprüchen gekennzeichnet.

Erfindungsgemäß ist erkannt worden, daß eine Ursache für Fehler bei Laserbehandlungen und insbesondere bei Laserkoagulationen ist, daß die Bedienungsperson bei der Behandlung, bei der sie aus einer Reihe von Gründen unter Zeitdruck steht, die zu behandelnde Stelle, die sie anhand bestimmter Strukturen wiederfinden soll, mit einem Bereich ähnlicher Struktur verwechselt.

Erfindungsgemäß ist deshalb vorgesehen, daß eine Bildverarbeitungseinrichtung dem auf dem Monitor dargestellten Bild des Auges ein zuvor aufgenommenes Bild dieses Auges überlagert, in dem die zu behandelnden Stellen markiert sind, daß die Steuereinheit der Bedienungsperson ermöglicht, das überlagerte Bild mit dem aufgenommenen Bild des Auges zur Deckung zu bringen; ein Bildsensor erfaßt das zu behandelnde Auge; die Steuereinheit führt aufgrund des Ausgangssignals des Bildsensors den Laser bei Bewegungen des Auges auf die in dem überlagerten Bild markierte Stelle nach.

Durch diese in dem zuvor aufgenommenen Bild vorhandene Markierung bzw. Markierungen (bei mehreren zu behandelnden Stellen) können Fehler vermieden werden, die beispielsweise durch das falsche Identifizieren von Strukturen hervorgerufen werden. Weiterhin führt die Steuereinheit das überlagerte und gegebenenfalls von der Steuereinheit vorpositionierte Bild, das die Bedienungsperson mit dem tatsächlichen Bild des zu behandelnden Auges, beispielsweise dem Fundus, zur Deckung gebracht hat, bei Bewegungen des Auges nach.

Damit muß die Bedienungsperson bei einem Behandlungsvorgang nur noch zu Beginn den Beobachtungsstrahlengang so justieren, daß der Beobachtungsstrahlengang in gleicher Weise wie bei dem zuvor aufgenommenen Bild verläuft. Hierdurch ergibt sich eine weitere Bedienungsvereinfachung, da die Bedienungsperson nur noch zu Beginn der Behandlung dafür sorgen muß, daß das eingespiegelte Bild mit der Vorlage des Auges zur Deckung gebracht ist:

Der Nachführgang kann dabei in der Weise erfolgen, wie dies beispielsweise gemäß der eingangs genannten Literaturstelle ARVO-Abstracts der Fall ist. Trotzdem hat man bei der erfindungsgemäßen Vorrichtung den Vorteil, daß der Arzt aufgrund des auf dem Monitor überlagerten Bildes sofort erkennt, wenn die Steuereinheit eine falsche Stelle als Referenz-Bereich identifiziert; damit kann der Arzt die Vorrichtung abschalten bzw. korrigieren.

Als weitere Hilfestellung für die Bedienungsperson kann die Steuereinheit den Laserstrahl so vorpositionieren, daß er auf die markierte Stellung gerichtet ist.

Durch die Kombination dieser Merkmale wird erreicht, daß der behandelnde Arzt in Ruhe vor Beginn der eigentlichen Behandlung einen Operationsplan erstellen kann, in dem er sich die zu behandelnden, beispielsweise die zu koagulierenden Stellen auf einem Bild des zu behandelnden Auges sucht und diese markiert. In dem Teil der Behandlung, in dem der Patient auf dem Operationsstuhl sitzt, und in dem erfahrungsgemäß schnell gearbeitet werden muß, positioniert die erfindungsgemäß vorgesehene Steuereinheit den Laserstrahl für den Arzt so vor, daß er sich nur noch von der Güte der Vorpositionierung überzeugen, eventuell notwendige Korrekturen durchführen und anschließend den Laserstrahl auslösen muß.

Andererseits wird durch die erfindungsgemäße Merkmalskombinations erreicht, daß die Bedienungsperson, also der behandelnde Arzt jederzeit die Güte der Positionierung des Laserstrahls anhand des auf dem Monitor zusätzlich überlagerten Bildes, das beispielsweise den gesamten Augenhintergrund wiedergibt, überprüfen kann. Da die Bedienungsperson "visuell" die Übereinstimmung des überlagerten Bildes mit dem tatsächlichen Bild über einen großen Bereich des Auges überprüfen kann, können "Laser-Fehlschüsse" nicht auftreten, die dadurch entstehen, daß fälschlicherweise zwei ähnlich aussehende Bereiche verwechselt und miteinander zur Deckung gebracht sind. Trotzdem ist bei der erfindungsgemäßen Vorrichtung lediglich ein geringer Rechenaufwand erforderlich, da beispielsweise nicht der gesamte, sehr stark strukturierte Augenhintergrund überprüft werden muß, sondern nur der Laserstrahl einer Markierung nachgeführt werden muß.

Selbstverständlich ist es auch möglich, den Laserstrahl zusätzlich manuell auszurichten (Anspruch 11), so daß der Arzt die Wahlfreiheit bei der Behandlung behält.

Mit der erfindungsgemäßen Vorrichtung ist es damit möglich, den Arzt von zeitaufwendigen und häufig von Hand nur ungenau durchzuführenden Vorgängen zu entlasten, ohne ihm die ärztliche Verantwortung bei der eigentlichen Behandlung zu nehmen.

Die erfindungsgemäße Vorrichtung ist in vorteilhafter Weise sowohl bei der Behandlung des Fundus als auch bei der Behandlung vorderer Augenabschnitte, wie der Cornea einsetzbar, und eignet sich zur Verwendung mit Behandlungslasern aller Wellenlängenbereiche, die im sichtbaren, im UV- oder im Infrarotbereich arbeiten. Auch ist es ohne weiteres möglich, mit einem gesonderten Ziellaser zu arbeiten.

Weiterbildungen der Erfindung sind in den Unteransprüchen gekennzeichnet.

Besonders vorteilhaft ist es, wenn in dem überlagerten Videobild (Anspruch 2) die zu behandelnde Stelle mittels Bildverarbeitung markiert ist; bei Videobildern kann dies leicht an einem entsprechenden Bildverarbeitungs-Eingabegerät erfolgen. Darüberhinaus kann das Videobild auf dem Monitor leicht beispielsweise mittels Hardware- oder Softwarescrolling nachgeführt und in der Größe angepaßt werden, ohne daß mechanische Verschiebungen von Optikelementen erforderlich wären.

Gemäß den Ansprüchen 3 und 4 weist die Steuereinheit einen Speicher auf, in dem die Behandlungsparameter, wie Ort der Koagulation oder des Schnitts in der Cornea, Leistung des Laserstrahls, Schußzeit etc. speicherbar sind. Diese Speicherung der Behandlungsparameter erlaubt nicht nur eine lückenlose Dokumentation, sondern ermöglicht auch eine Auswertung der mit der erfindungsgemäßen Vorrichtung durchgeführten Behandlungsvorgänge in wissenschaftlicher und/oder rechtlicher Hinsicht. Dabei ist es in jedem Falle vorteilhaft, wenn die Steuereinheit ein Ausgabegerät, beispielsweise einen Protokolldrucker aufweist, mit dem die Soll- und Ist-Behandlungsparameter ausgegeben werden (Anspruch 5).

Besonders vorteilhaft ist es, wenn sowohl die für die Behandlung vorgegebenen Parameter als auch die tatsächlichen Behandlungsparameter auf dem Monitor erkennbar sind (Anspruch 6), wobei es insbesondere vorteilhaft ist, wenn eine Zuordnung der Parameter zu der jeweiligen Markierung erfolgt (Anspruch 7).

Die Steuereinheit kann in Falle auch dazu verwendet werden, verschiedene Sicherungsfunktionen auszuführen:

Gemäß Anspruch 8 ist vorgesehen, daß die Steuereinheit den Behandlungsvorgang unterbricht, wenn das projizierte Bild nicht mehr ausreichend mit dem Auge in Deckung ist.

Eine Abschaltung des Laserstrahls kann auch erfolgen, wenn die Weißverfärbung des Fundus eine bestimmte Stufe erreicht hat (Anspruch 9) oder eine bestimmte Energie auf der zu behandelnden Stelle deponiert ist (Anspruch 10).

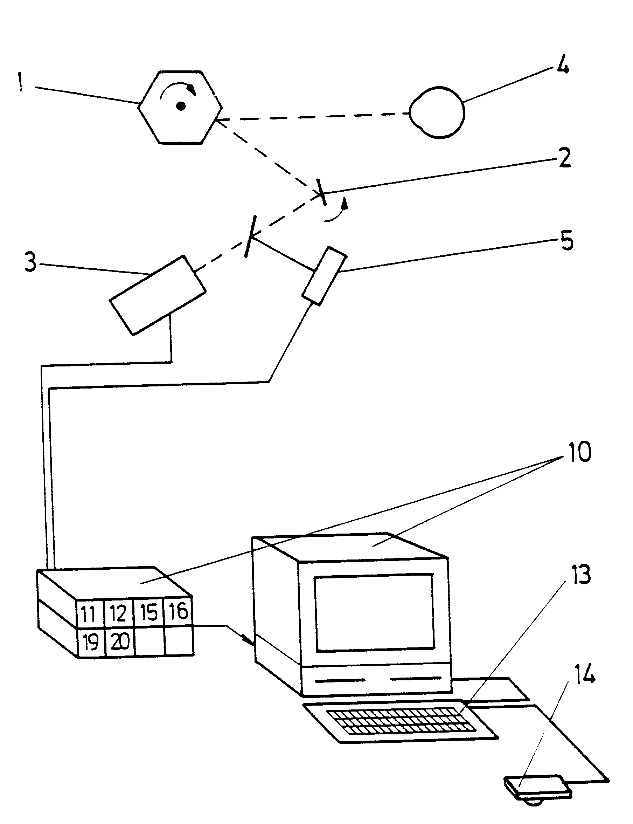

## Kurze Beschreibung der Zeichnung

Die Erfindung wird nachstehend anhand eines Ausführungsbeispiels unter Bezugnahme auf die Zeichnung näher beschrieben,

deren einzige Figur schematisch eine erfindungsgemäße Vorrichtung zeigt.

## Darstellung eines Ausführungsbeispiels

Die erfindungsgemäße Vorrichtung weist ein Laser-Scanning-Ophthalmoskop mit einer Abtasteinrichtung auf, die bevorzugt aus einem x/y-Scanner mit einer Polygon-Spiegeltrommel (1) und einem Galvanometerspiegel (2) besteht, da ein derartiges System wellenlängenunabhängig arbeitet, was insbesondere bei der gleichzeitigen Verwendung von Licht mehrerer Wellenlängen von Vorteil ist.

Die Abtasteinrichtung richtet den Strahl eines Lasers (3) derart auf ein zu untersuchendes und zu behandelndes Auge (4), daß der Laserstrahl u.a. den zu behandelnden Bereich abtastet. Das von dem Auge reflektierte Licht wird bei dem gezeigten Ausführungsbeispiel über die Abtasteinrichtung (1, 2) auf eine Detektoreinrichtung (5) geleitet, deren zeitsequentielle Ausgangssignale eine elektronische Steuer- und Auswerteeinheit mit an sich bekanntem Aufbau synchronisiert zur Abtastbewegung u.a. an einen Videomonitor (6) anlegt, auf dem damit ein Bild des Auges sichtbar wird.

Die nur schematisch dargestelle Steuereinheit (10) weist einen zur Bildverarbeitung geeigneten Rechner (11), einen Steuermonitor (12), eine Tastatur (13), die gegebenenfalls auch eine Maus-Eingabe (14) aufweisen kann, ein Anzeigepanel (15), eine Bildspeichereinheit (16), einen Bildsensor (17), der das zu behandelnde Auge (4) detektiert, und einen Bildmonitor (18) sowie einen externen Speicher (19) beispielsweise ein Diskettenlaufwerk und einen Drucker (20) auf.

Die Steuereinheit (10) steuert nicht nur die Leistung und die Schußzeit des Lasers, sondern auch über den Strahlmanipulator (8) den Auftreffort des Laserstrahls im Auge derart, daß er mit einer im eingespiegelten Bild vorgesehenen Markierung übereinstimmt. Ferner führt die Steuereinheit (10) das eingespiegelte Bild, das die Bedienungsperson mit dem Auge zur Deckung gebracht hat, bei Bewegungen des zu untersuchenden Auges nach.

Die Steuereinheit erlaubt ferner die Manipulation eines zuvor mit dieser Vorrichtung oder mit einem anderen Untersuchungsgerät aufgenommenen Bildes vor dem eigentlichen Behandlungsvorgang. Damit kann beispielsweise ein Chefarzt die Behandlung - Koagulations- bzw. Schnittort, deponierte Energie etc. - planen, die eigentliche Behandlung aber einem Assistenten überlassen.

Vorstehend ist die Erfindung anhand eines Ausführungsbeispiels beschrieben worden. Innerhalb des allgemeinen Erfindungsgedankens sind natürlich die verschiedensten Modifikationen möglich:

Die Steuereinheit kann auch die Regelung anderer Größen übernehmen, z.B. kann sie die deponierte Energie gemäß den in der DE-OS 30 24 169 oder der DE-OS 33 06 981 beschriebenen Algorithmen regeln.

Besonders vorteilhaft ist es jedoch in jedem Falle, wenn der Strahl eines Koagulationslaser, also beispielsweise eines $Ar^+$-Lasers oder eines Farbstofflasers zwischen Abtasteinrichtung und Auge eingespiegelt wird. Natürlich ist es aber auch möglich, zum Koagulieren die Leistung eines Beobachtungslasers "kurzfristig" zu erhöhen, wie dies in der US-PS 4 213 678 beschrieben ist. Die Mitverwendung der "Scan-Einrichtung" ermöglicht in diesem Falle insbesondere die Behandlung größerer Gebiete bzw. mehrerer Gebiete in einem Schritt.

Eine Vorrichtung zur Beobachtung der hinteren Augenabschnitte mit abtastender Beleuchtung ist wegen der reflexfreien und hochauflösenden Bilddarstellung besonders als Bildgeber für eine sog. Eye-Tracking-Einheit prädestiniert.

## Patentansprüche

1. Vorrichtung zur Beobachtung und Laserbehandlung des Auges,
mit einer Beleuchtungs-Lichtquelle, deren Licht ein optisches System auf den zu beobachtenden Teil des Auges über eine Abtasteinrichtung (1, 2) fokussiert, die eine Abtastbewegung des Lichts auf dem beobachtenden Teil des Auges (4) erzeugt,
mit einer Detektoreinrichtung (5), die das an dem zu beobachtenden Teil des Auges (4) reflektierte Licht empfängt, und deren Ausgangssignal an eine Auswerte- und Synchronisiereinheit angelegt ist, die aus dem zeitsequentiellen Ausgangssignal der Detektoreinrichtung ein Bild des beobachteten Teil des Auges (4) erzeugt, und deren Ausgangssignal an einem Monitor (6) zur Darstellung dieses Bildes anliegt,
und mit einer Behandlungs-Lasereinrichtung (7), deren Laserstrahl eine Steuereinheit (10) mittels einer Manipulatoreinheit (8) auf den zu behandelnden Bereich des Auges (4) ausrichtet,
dadurch **gekennzeichnet**, daß eine Bildverarbeitungseinrichtung (11) dem auf dem Monitor (6) dargestellten Bild des Auges ein zuvor aufgenommenes Bild dieses Auges überlagert, in dem die zu behandelnden Stellen markiert sind, daß die Steuereinheit der Bedienungsperson ermöglicht, das überlagerte Bild mit dem aufgenommenen Bild des Auges zur Deckung zu bringen,
und daß ein Bildsensor (17) das zu behandelnde Auge erfaßt, und die Steuereinheit (10) aufgrund des Ausgangssignals des Bildsensors den Laser bei Bewegungen des Auges auf die in dem überlagerten Bild markierte Stelle nachführt.

2. Vorrichtung nach Anspruch 1,
dadurch **gekennzeichnet**, daß die zu behandelnde Stelle in dem eingespiegelten Videobild mittels Bildverarbeitung markiert ist.

3. Vorrichtung nach Anspruch 1 oder 2,
dadurch **gekennzeichnet**, daß die Steuereinheit einen Speicher aufweist, in dem die Parameter für die Behandlung speicherbar sind.

4. Vorrichtung nach Anspruch 3,
dadurch **gekennzeichnet**, daß in dem Speicher die tatsächlichen Behandlungsparameter speicherbar sind.

5. Vorrichtung nach Anspruch 3 oder 4,
dadurch **gekennzeichnet**, daß die Steuereinheit eine Ausgabeeinheit (20) aufweist.

6. Vorrichtung nach einem der Ansprüche 3 bis 5,
dadurch **gekennzeichnet**, daß die Soll- und/oder Ist-Behandlungsparameter auf dem Monitor eingespiegelt sind.

7. Vorrichtung nach einem der Ansprüche 1 bis 6,
dadurch **gekennzeichnet**, daß die Behandlungsparameter aus der Markierung im überlagerten Bild erkennbar sind.

8. Vorrichtung nach einem der Ansprüche 1 bis 7,
dadurch **gekennzeichnet**, daß die Steuereinheit die Behandlung unterbricht, wenn das überlagerte Bild ungenügend mit dem Bild des Auges zur Deckung gebracht ist.

9. Vorrichtung nach einem der Ansprüche 1 bis 8,
dadurch **gekennzeichnet**, daß die Steuerschaltung einen Detektor aufweist, der die Weißverfärbung des Koagulats erfaßt.

10. Vorrichtung nach Anspruch 9,
dadurch **gekennzeichnet**, daß die Steuerschaltung die vom Laser deponierte Energie regelt.

11. Vorrichtung nach einem der Ansprüche 1 bis 10,
dadurch **gekennzeichnet**, daß der Laserstrahl zusätzlich manuell ausrichtbar ist.

## Claims

1. A device for the observation and laser treatment of the eye,
with an illumination light source whose light is focused by an optical system onto the portion of the eye to be observed by a scanning unit (1,2) which generates a scanning movement of the light on the portion of the eye to be observed (4),
with a detector unit (5) which receives the light reflected on the portion of the eye to be observed (4) and whose output signal is transmitted to an evaluation and synchronization unit which generates an image of the observed portion of the eye (4) from the time sequential output signal of the detector unit and whose output signal is transmitted to a monitor (6) for the display of the image,
and with a treatment laser unit (7) whose laser beam is aligned to the area of the eye to be treated (4) by a control unit (10) by means of a manipulator unit (8),
characterized by the fact that an image processing unit (11) superimposes onto the image of the eye displayed on the monitor (6) a previously taken image of this eye in which the areas to be treated are marked, that the control unit permits the operator to bring the superimposed image flush with the taken image of the eye,
and that an image sensor (17) registers the eye to be treated and the control unit realigns the laser on the basis of the output signal of the image sensor to the area marked in the superimposed image when the eye moves.

2. A device according to Claim 1,
**characterized** by the fact that the area to be treated is marked in the reflected video image by means of image processing.

3. A device according to Claims 1 or 2,
**characterized** by the fact that the control unit possesses a memory in which the parameters for the treatment can be stored.

4. A device according to Claim 3,
**characterized** by the fact that the actual treatment parameters can be stored in the memory.

5. A device according to Claims 3 or 4,
**characterized** by the fact that the control unit possesses an output unit (20).

6. A device according to any of Claims 3 to 5,
**characterized** by the fact that the nominal and/or actual treatment parameters are displayed on the monitor.

7. A device according to any of Claims 1 to 6,
**characterized** by the fact that the treatment parameters can be recognized from the marking in the superimposed image.

8. A device according to any of Claims 1 to 7,
**characterized** by the fact that the control unit interrupts the treatment if the superimposed image is insufficiently flush with the image of the eye.

9. A device according to any of Claims 1 to 8,
**characterized** by the fact that the control circuit possesses a detector which registers the white discoloration of the coagulate.

10. A device according to Claim 9,
**characterized** by the fact that the control circuit regulates the energy discharged by the laser.

11. A device according to any of Claims 1 to 10,
**characterized** by the fact that the laser beam can additionally be aligned manually.

**Revendications**

1. Installation à laser pour l'examen et le traitement de l'oeil, comprenant
une source lumineuse d'éclairement, dont un système optique concentre la lumière sur la partie de l'oeil à examiner, par l'entremise d'un dispositif analyseur (1, 2) qui provoque un mouvement de balayage de la lumière sur cette partie de l'oeil,
un dispositif détecteur (5), qui reçoit la lumière réfléchie sur la partie examinée de l'oeil (4) et dont le signal de sortie est appliqué à un circuit d'exploration et de synchronisation qui produit, à partir du signal récurrent de sortie de ce dispositif détecteur une image de la partie examinée de l'oeil et dont le signal de sortie est appliqué à un moniteur (6) destiné à reproduire cette image, et
une installation (7) de traitement au laser, dont un dispositif (10) de commande dirige au moyen d'un appareil manipulateur (8) le rayon sur la partie à traiter de l'oeil,
installation caractérisée en ce qu'un dispositif (11) de traitement des images superpose, à l'image de l'oeil reproduite sur le moniteur (6), une image de cet oeil prise antérieurement, sur laquelle les endroits à traiter sont repérés,
le dispositif de commande permet à l'opérateur de faire coïncider l'image superposée avec l'image prise de l'oeil, et
un capteur (17) d'images détecte l'oeil à traiter et le dispositif (10) de commande fait suivre au laser, sous l'effet du signal de sortie de ce capteur d'images, l'endroit repéré sur l'image superposée, quand l'oeil bouge.

2. Installation selon la revendication 1,
caractérisée en ce que l'endroit à traiter (médicalement) est marqué par traitement (électronique) de cette image, par un repère sur l'image vidéo réfléchie.

3. Installation selon la revendication 1 ou 2,
caractérisée en ce que le dispositif de commande comporte une mémoire dans laquelle les paramètres du traitement peuvent être enregistrés.

4. Installation selon la revendication 3,
caractérisée en ce que les paramètres effectifs du traitement peuvent être enregistrés dans la mémoire.

5. Installation selon la revendication 3 ou 4,
caractérisée en ce que le dispositif de commande comporte un dispositif (20) de sortie.

6. Installation selon l'une des revendications 3 à 5,
caractérisée en ce que les paramètres de consigne et/ou réels du traitement sont reproduits sur le moniteur.

7. Installation selon l'une des revendications 1 à 6,
caractérisée en ce que les paramètres du traitement (médical) peuvent être déterminés d'après les repères sur l'image superposée.

8. Installation selon l'une des revendications 1 à 7,
caractérisée en ce que le dispositif de commande interrompt le traitement (médical) si l'image superposée n'est pas amenée à coïncider suffisamment avec l'image de l'oeil.

9. Installation selon l'une des revendications 1 à 8,
caractérisée en ce que le dispositif de commande comporte un détecteur qui détecte le blanchiment du coagulat.

10. Installation selon la revendication 9,
caractérisée en ce que le dispositif de commande règle la quantité d'énergie déposée ou appliquée par le laser.

11. Installation selon l'une des revendications 1 à 10,
caractérisée en ce que le rayon laser peut aussi être orienté manuellement.